# EUROPEAN PATENT APPLICATION

(11) **EP 2 962 683 A1**
(43) Date of publication of application: **06.01.2016**
(21) Application number: 15180762.5
(22) Date of filing: 17.05.2004
(51) Int. Cl.: A61K 9/08, A61P 13/12, A61M 1/28

(54) **LOW SODIUM SOLUTION**

(30) Priority: 28.05.2003 SE 0301577; 19.11.2003 US 523722 P
(62) Divisional of application: 04733508.8
(71) Applicant: Fresenius Medical Care Deutschland GmbH, 61352 Bad Homburg (DE)
(72) Inventor: Wieslander, Anders, S-227 38 Lund (SE); Carlsson, Ola, S-226 49 Lund (SE); Bernard, Pascal, F-69130 Ecully (FR); Ulinder, Per, S-254 50 Helsingborg (SE)
(74) Representative: Behr, Wolfgang

(57) **Abstract**

The present application shows a container for preparation of a peritoneal dialysis solution, the container comprising a first compartment containing a first solution comprising sodium ions and at least a second compartment containing a second solution comprising glucose, wherein mixing of the first solution with the at least second solution results in a final solution comprising sodium ions in a concentration of 100 to 115 mM, glucose in a concentration of 1% to 5% by weight, and a low level of glucose degradation products.

## Description

### Background of the Invention

The present invention relates to a medical solution, a method for preparing the medical solution, a container for preparation of the solution, use of said solution for manufacture of a medicament for treatment of dialysis, and a method of treatment of dialysis with said solution.

### Background Art

Peritoneal dialysis is a method for exchanging solutes and water in capillary vessels of a patient's peritoneum with hypertonic solution, which is infused into the peritoneal cavity. The principle of this method is diffusion of solutes transported according to the concentration gradient and water migration due to osmotic differences. This method has many advantages, e.g. no special apparatus is commonly required. It gives less influence on the hemodynamics because extracorporeal circulation of the patient's blood is not necessary, and further the peritoneal dialysis is a continuous treatment and therefor more similar to the function of the kidneys.

Peritoneal dialysis is usually classified as continuous ambulatory peritoneal dialysis (CAPD), intermittent peritoneal dialysis (IPD), continuous cyclic peritoneal dialysis (CCPD) or automated peritoneal dialysis (APD).

In peritoneal dialysis, a catheter is permanently implanted in the abdominal wall of the patient and about 1.5 to 2.5 1 of the dialysis fluid is normally introduced via the catheter into the peritoneal cavity. The peritoneal cavity is flooded with this fluid, left for an appropriate lapse of time and then drained. Removal of solutes and water takes place across the peritoneum, which acts as a semipermeable membrane.

The dialysis fluid normally used for peritoneal dialysis is an aqueous solution comprising an osmotic agent such as glucose and the like, electrolytes such as sodium, potassium, calcium, magnesium, and organic acid salts such as sodium lactate, sodium bicarbonate, or sodium pyruvate. The components of these peritoneal dialysis fluids are selected to control the levels of electrolytes or the acid-base equilibrium, to remove waste materials and to efficiently carry out ultrafiltration.

It is known to pack medical solutions in multicompartment bags from e.g. WO 99/27885 (Gambro Lundia AB), in which different solutes may be kept in separate compartments of the bag with a view to, inter alia, regulating the concentration of active ingredients in the finally prepared solution.

Peritoneal dialysis patients tend to have disturbances in their sodium and water balance. To correct this, it has previously been necessary to increase the glucose concentration in the dialysate in order to remove enough water from the patient. Since glucose load in peritoneal dialysis patients is a burden on their nutritional status, it is important to reduce the glucose load as much as possible. It has previously been reported that a sodium excess in continuous ambulatory peritoneal dialysis patients with water overload could be treated by using dialysis solutions with very low concentrations of sodium (Nakayama N, Yokoyama K, Kubo H, Watanabe S, Kawaguchi Y, Sakai O: Effects of ultra low Na concentration dialysate (ULNaD) for overhydrated patients undergoing CAPD. Perit Dial Int 12(Suppl 1):143, 1992). A low sodium concentration results in higher sodium extraction, which is important for patients with a fluid overload.

Passlich-Deetjen J. et al., Solutions for APD: Special Considerations. Seminars in Dialysis, 15(6) 2002, 407-413, discloses automated peritoneal dialysis solutions containing sodium and glucose and having reduced amounts of GDPs.

EP-A2-0 958 832 discloses an albumin containing peritoneal dialysis fluid also containing sodium and glucose.

EP-A1-1 008 341 discloses a glucose-containing preparation for peritoneal perfusion also containing sodium and having an almost neutral pH.

Nakayama M. et al., Effects of Ultra Low Na Concentration Dialysate (ULNaD) for Overhydrated Patients undergoing CAPD. Peritoneal Dialysis International 12, 1992, Suppl. 1, 195, discloses a dialysis liquid containing sodium and glucose.

Problems with fluid overload and inadequate sodium removal usually appear in patients after some time on PD when negative effects from non-biocompatible fluids appear.

It has been shown in clinical studies that a low sodium concentration gives a reduced fluid transport (Ultrafiltration, UF) if the osmolality of the fluid is uncorrected, i.e. with an increased glucose concentration (Amici G. et al. Low sodium concentration solution in normohydrated CAPD patients. Adv. Perit. Dial. 11:78-82, 1995).

Many studies on cells from the peritoneal membrane have demonstrated adverse effects from peritoneal dialysis fluids. The most obvious aspects are low pH, high lactate concentration, high osmolality and high glucose concentration. However, a major problem arises when it comes to production of fluids of this type for clinical use, since they must be sterile. Sterilisation is normally performed by the addition of energy, i.e. heat. It is known that when heat sterilising carbohydrates, e.g. glucose, some of the glucose is degraded to reactive substances called Glucose Degradation Products (GDP), which may be higly reactive aldehydes (Nilsson-Thorel CB, Muscalu N, Andren AH, Kjellstrand PT, and Wieslander AP: Heat sterilisation of fluids for peritoneal dialysis gives rise to aldehydes. Perit. Dial. Int. 13(3):208-213, 1993; and EP-B1 0 668 785). These aldehydes are not only known to be cytotoxic but also to severely accelerate AGE (advanced glycation end product) formation. The chemical nature of the GDP in peritoneal dialysis fluids is not yet fully understood. One of the best known GDP is 5-HMF (5-Hydroxymethylfuraldehyde) and various pharmacopoeias restrict the concentration of 5-HMF in medical fluids. The limits that are set are, however, often much higher than those found in peritoneal dialysis fluids. Other identified and quantified GDPs in peritoneal dialysis fluids are formaldehyde, acetaldehyde, methylgloxal, glyoxal, 2-furaldehyde, 3-deoxyglucosone (3-DG) and 3,4-dideoxyglucosone (3,4-DGE). However, these identified GDPs only represent a fraction of degradation products that can be generated from glucose. Thus, there has been a long-felt need to solve the above defined problems and to provide a biocompatible sterilised medical solution, in particular a dialysis solution, containing an optimal mixture of sodium and glucose.

It has been shown that a biocompatible PD solution, based on lactate as buffer, but containing low levels of GDP and a pH close to neutral, can preserve the peritoneal membrane of rats. Table 1 demonstrates that the preservation of the membrane was after three months manifested as higher ultrafiltration capacity in rats exposed to a low GDP fluid (Gambrosol™trio) compared to rats exposed to a conventional PD fluid, with acidic pH and high concentration of GDP (Carlsson O et al, Preserved ultra-filtration with a PD solution containing less glucose degradation products after 3 months of intraperitoneal injections in rats. Perit. Dial. Int. 21(Suppl. 2): S145, 2001).

It has also in clinical studies been demonstrated that pH neutral PD fluids containing bicarbonate (and reduced concentration of GDP) may give a raised UF volume (Tranæus A: A long-term study of a bicarbonate/lactate-based peritoneal dialysis solution-clinical benefits. Perit. Dial. Int. 20(5):516-523, 2000). But on the other hand, there are examples of reduced UF volumes as well when using other biocompatible PD fluids containing bicarbonate (Montenegro J. et al Peritoneal transport with 3 different peritoneal solutions. Nephrol. Dial. Transplant. 18(Suppl. 4):216 2003). The reason for the different results is not quite clear, but the bicarbonate buffer may have negative effects on fluid transport while low GDP levels have a positive effect. Especially since rat experiments have shown that low GDP levels in lactate buffered solutions give a positive effect on fluid transport (Musi B. et al Biocompatibility of peritoneal dialysis fluids: Long-term exposure of nonuremic rats. Perit. Dial. Int. 24(1):37-47, 2004). The most important difference between the two clinical studies and the rat study cited above is that the clinical studies were performed on patients that have been on PD for some time, using conventional PD fluids, prior to the study while the rats were untreated prior to the study. The treatment with conventional fluids probably has already damaged the peritoneal membrane in those patients and, therefor, the positive effect from a biocompatible solution may not be seen. Furthermore, it is likely that the rats give a clearer view of the effects of GDPs on the transport across the peritoneal membrane since they were untreated prior to the study.

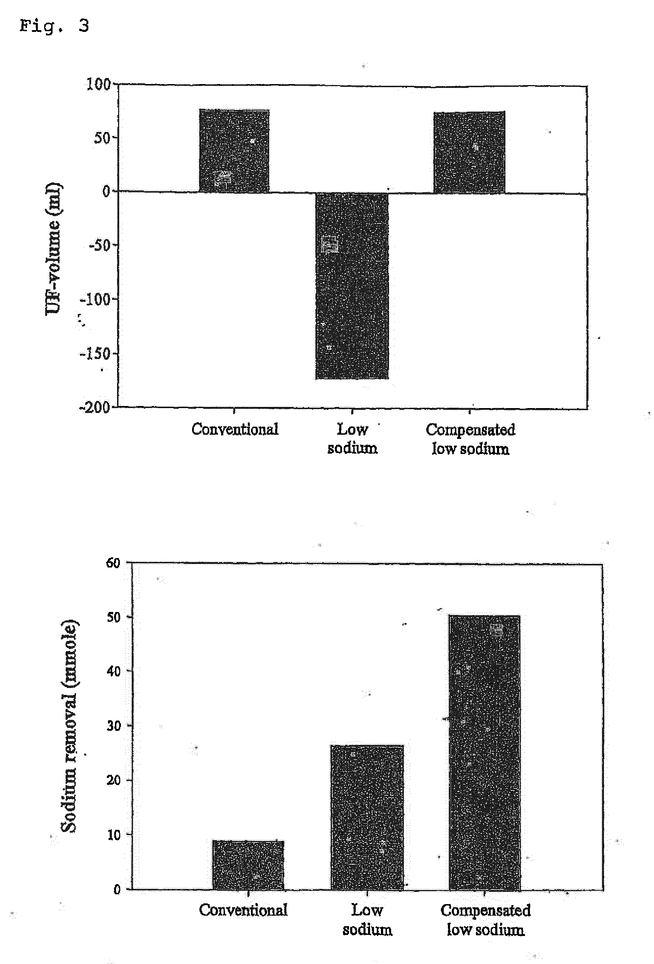

Furthermore, it can be shown from computer simulations that it is important to maintain the UF volume to get an increased sodium extraction (Fig. 3). The first panel shows simulated UF volume in an average patient during.a 4-hour dwell, wherein (Conventional) indicate dialysis using a conventional PD fluid containing 1.5% glucose and 132 mM sodium, (Low sodium) a solution containing 1.5% glucose and 102 mM, and (Compensated low sodium) a solution containing 2.5% glucose and 102 mM sodium. The first panel shows that the UF volume is dependent on both the sodium and the glucose concentration. The second panel shows sodium removal from the same set of simulations as above. When a low sodium concentration is used, it is important to have a high UF volume as in compensated low sodium where the reduced osmolality due to decreased sodium chloride concentration has been compensated for by an increased glucose concentration, which attenuates the sodium removal.

Thus, if a biocompatible solution with low GDP and a pH above 6.5 maintains high ultrafiltration over time and in addition contains lower levels of sodium, it is possible to increase the efficiency, and to lever the sodium removal, compared to a conventional non-biocompatible low sodium solution.

### Summary of the Invention

The object of the present invention is to solve the above-mentioned problem.

According to the present invention, this object is achieved by a medical solution comprising sodium ions in a concentration of 90-125 mM, glucose in a concentration of 1-5% by weight, and a low level of glucose degradation products, wherein said solution is sterile and has a pH of 6.5-8.0.

Further, the invention relates to a method for preparing said solution.

The present invention also relates to a container for preparation of the solution.

In another aspect, the invention relates to the solution according to the invention for use as a medicament.

In a further aspect, the invention relates to use of said solution for manufacture of a medicament for dialysis.

In still another aspect, the present invention relates to a method for treatment of dialysis, said method comprising administering of the solution according to the invention to a patient having a need therefor.

Further disclosure of the objects, problems, solutions and features of the present invention will be apparent from the following detailed description of the invention with reference to the drawings and the appended claims.

### Brief Description of the Drawings

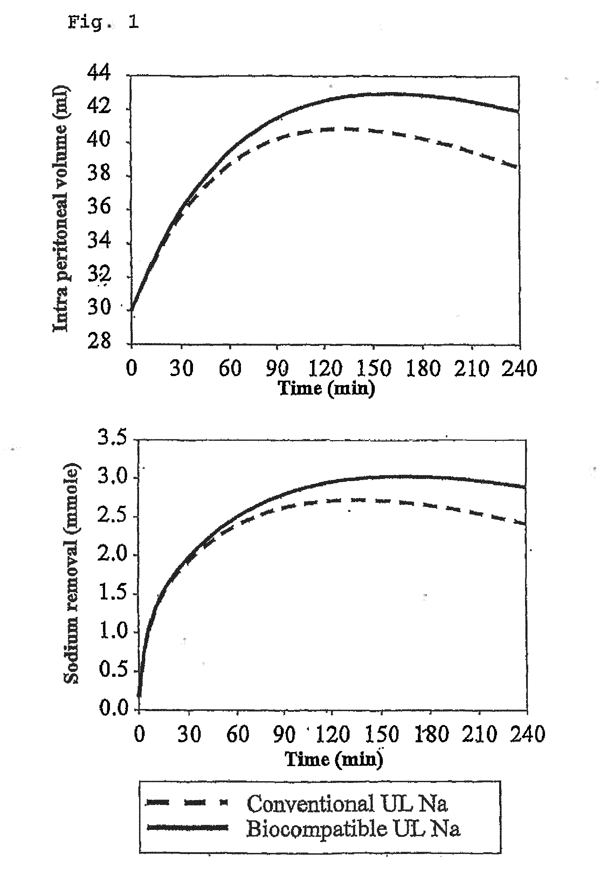

Fig. 1 is a graph showing computer simulated fluid (upper diagram) and sodium removal (after three months of peritoneal exposure) with time for a conventional low sodium solution and for a lactate based biocompatible (low GDP and near a neutral pH) low sodium solution according to a preferred embodiment of the invention. Simulations have been performed based on data from the above-mentioned study by Musi et al. The computer model is based on the three-pore model of peritoneal transport.

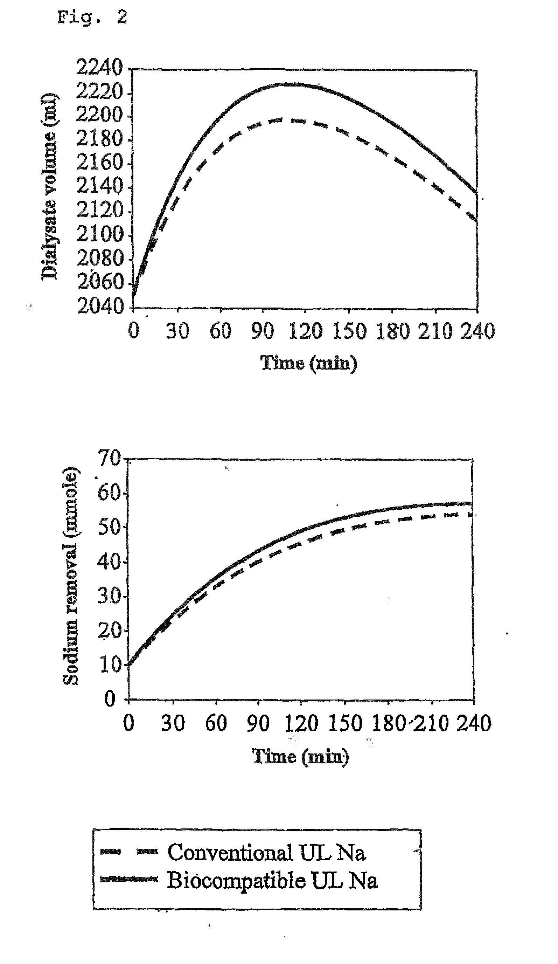

Fig. 2 is a graph (upper diagram) showing computer simulations of the dialysate volume with time for a conventional low sodium solution and for a biocompatible low sodium solution containing bicarbonate according to the invention, as well as a graph (lower diagram) showing the sodium removal with time for a conventional low sodium solution and for a biocompatible low sodium solution containing bicarbonate according to a preferred embodiment of the invention.
Fig. 3 describes computer simulations using human data. The upper panel shows UF volume after 240 min dwell time using either a 1.5% glucose solution with 132 mM sodium (conventional), a low sodium solution containing 1.5% glucose and 102 mM sodium, or a low sodium solution with the osmolality corrected to the same level as the conventional solution i.e. 2.5% glucose and 102 mM sodium.

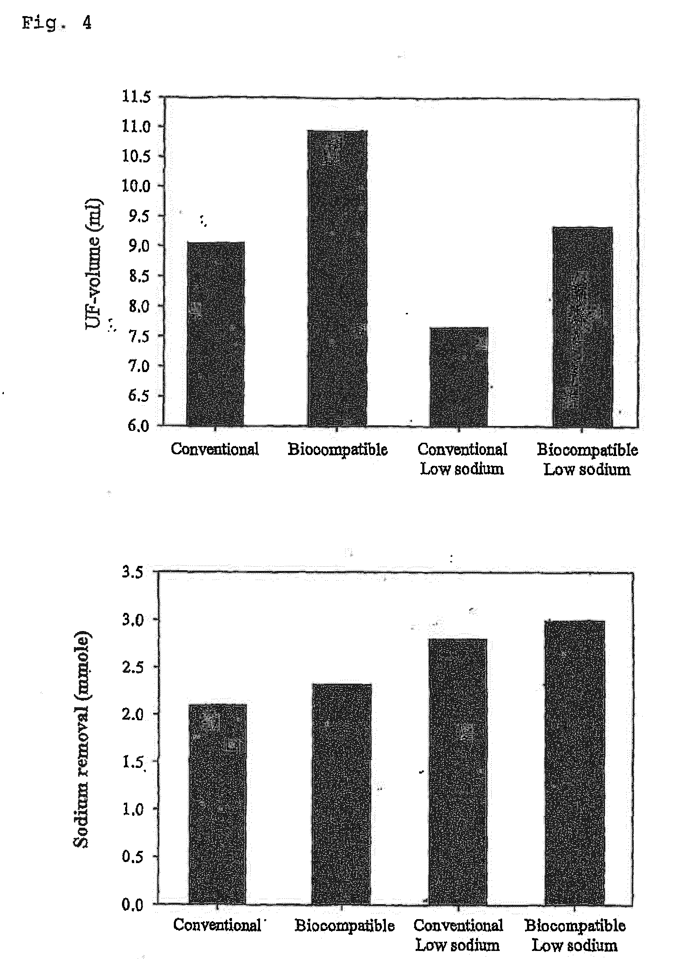

Fig. 4 describes the results from a computer simulation for rats where input data from a study by Musi et al. have been used (Musi B. et al Biocompatibility of peritoneal dialysis fluids: Long-term exposure of nonuremic rats. Perit. Dial. Int. 24(1):37-47, 2004) where rats were treated for 12 weeks with either a conventional solution containing GDPs or with a low GDP solution (Gambrosol trio). Data from those two groups were used in the simulation (A₀/Δₓ and LₚS) to test the effect of long-term treatment with different GDP content. The first and second bar represent simulations using a 3.9% glucose solution with 132 mM sodium, while the third and fourth bar represent simulations with a solution containing 3.9% glucose and 102 mM sodium. The upper panel shows UF volume and the lower panel shows sodium removal.

### Detailed flescri tion of Different Embodiments

The container used according to one embodiment of the present invention is based on the multicompartment bag disclosed in WO 99/27885 (Gambro AB), in which different solutes may be kept in separate compartments of the bag with a view to, inter alia, regulating the concentration of active ingredients in the finally prepared peritoneal dialysis solution.

The container in WO 99/27885 thus comprises a large compartment containing sodium bicarbonate, sodium lactate and sodium chloride, as well as a plurality of small compartments containing e.g. calcium ions, sodium ions, chloride ions, and glucose. The container is sterilised in an autoclave with the solutions in situ in said compartments. One or more of the small compartments are connected to the large compartment by frangible pins or peelable seals, whereby the contents of the compartments may be mixed and the peritoneal dialysis solution is obtained.

Turning now to the present application, Fig. 1 shows computer simulations of intraperitoneal volume and sodium removal from rats treated with conventional and biocompatible peritoneal dialysis solutions respectively during three months. The simulation is performed for a 600 g rat treated with a PD solution containing 3.9% glucose and 102 mM sodium. Since the UF volume is preserved in animals treated with a biocompatible solution, the effect from a low sodium solution is more pronounced in this case compared to animals treated with conventional solutions. More precisely, from the graphs in Figs 1 and 2, it can be seen that the sodium removal with time for a biocompatible, with or without bicarbonate, low sodium solution according to a preferred embodiment of the invention is more pronounced than the sodium removal with time for a conventional low sodium solution. Thus, the sodium overload decreases more efficiently with a biocompatible low sodium solution according to the invention than with a conventional low sodium solution. The concentration of sodium, for both solutions, is in this embodiment 102 mM.

From the graphs in Figs 1 and 2 it can also be seen that the dialysate volume is larger for a biocompatible, with or without bicarbonate, low sodium solution according to one embodiment of the invention than for a conventional low sodium solution. The concentration of sodium for these solutions is also 102 mM. The term "UL Na" in connection with Figs 1 and 2 means ultra low sodium content.

From the graph in Fig. 3 it can be seen from the upper panel that the UF volume is significantly affected by the reduction of the sodium concentration in the low sodium group and the lower panel shows that the sodium removal is increased. If, however, the reduced osmolality is corrected by an increase in glucose, the UF volume is maintained at the same level as in a conventional solution and the sodium removal is even higher than in the low sodium group.

From the graph in Fig. 4 it is clear from those graphs that it is important to keep the UF volume as high as possible to increase the sodium removal and maintain as much as possible of the UF volume.

Table 1 below demonstrates the preserved ultrafiltration capacity after long-term exposure to a PD fluid with low levels of GDP and a near neutral pH.

### Table 1

The long-term effect from a conventional solution and a solution containing low levels of GDP was compared with untreated controls, and the following results were obtained:

| | UF volume±SEM |
|---|---|
| Untreated control | 12.32±0.72 ml |
| Conventional solution | 8.87±1.13 ml |
| Biocompatible solution | 12.12±1.18 ml |

The medical reasons behind these advantageous results are at present not known. One might however speculate that GDPs could be the reason behind some of the long-term changes observed for the peritoneal membrane, such as ultrafiltration failure. Accumulation of AGE in the peritoneal membrane has been connected to the development of ultrafiltration failure for PD patients. As GDP is one of the strongest promoters of AGE formation in the fluid, there is a clear indirect link between ultrafiltration failure and GDP.

Bicarbonate alone, or in combination with reduction of GDP, can also in the short-term increase ultrafiltration, presumably the result of the neutral pH inducing less vasodilatation of the capillaries.

A biocompatible low sodium solution according to the present invention, comprising sodium ions in a concentration of 90-125 mM, glucose in a concentration of 1-5% by weight, and a low level of glucose degradation products, wherein said solution is sterile and has a pH of 6.5-8.0, is prepared in a multicompartment container, e.g. according to WO 99/27885. A solution comprising sodium ions is thus provided in a first compartment of the container and a solution comprising glucose is provided in at least one further compartment that is delimited from the first compartment during sterilisation of the container and its contents. The whole container may thus be heat sterilised with the solutions in situ in said compartments.

The sterilisation is, for instance, heat sterilisation effected in an autoclave at a temperature of at least 100°C, e.g. above 120°C. The sterilisation time may vary depending on the sterilisation temperature, the type of container and the contents therein to be sterilised.

The sterilisation can, however, also be effected for separated interconnectable containers comprising the solutions to be sterilised and provided with connection means with sterile connecting valves for sterile connection.

After sterilisation, the contents of the first compartment may be mixed with the contents of at least one of said further compartments to form a biocompatible solution with the characteristics as stated above.

The prepared medical solution according to the invention comprises in one embodiment a concentration of sodium ions of 100-115 mM. Further, a prepared medical solution according to the invention comprises in one embodiment a glucose concentration of 1.5-4% by weight. Even further, in another embodiment a prepared medical solution according to the invention has a pH of 7.0-7.8.

In another embodiment the medical solution, after mixing, comprises a concentration of sodium ions of 102 mM, a glucose concentration of either 1.5%, 2.5% or 3.9% by weight and a pH of 7.4. In still another embodiment the medical solution contains, after mixing, a sodium concentration of 102-115 mM and a glucose concentration of 2.0%, 2.5%, or 4.3% by weight and a pH of 7.4.

In one embodiment of the present invention the medical solution, after mixing, also comprises bicarbonate at a concentration of 5 mM to 45 mM, e.g. 25 mM to 40 mM, or lactate at a concentration of 5 mM to 45 mM, e.g. 25 mM to 40 mM, or a combination of both where the total concentration of bicarbonate and lactate does not exceed 45 mM, e.g. a concentration of lactate of 10 mM and a concentration of bicarbonate of 30 mM.

In still another embodiment of the invention, the medical solution also comprises other electrolytes, e.g. one or more of potassium, calcium and magnesium.

In one embodiment of the present invention, the container used for the method of preparing the medical solution according to the invention comprises two compartments, i.e. a first compartment comprising sodium ions and a second compartment comprising glucose.

In another embodiment, the container used for the preparing of the medical solution comprises three compartments, i.e. a first compartment comprising sodium ions and two compartments comprising glucose. The glucose concentration in at least one further compartment is provided to be above 10%, e.g. above 20%, such as above 40%, by weight. Moreover, the pH in the at least one further compartment including glucose is 2-5. Further, sodium ions may also be provided in the at least one further compartment containing glucose.

In one embodiment, the first compartment of the container further also contains bicarbonate ions and/or lactate.

The container used according to the present invention may also contain one or more further compartments in addition to the three compartments mentioned above, if desired.

The volume of each compartment, as well as the proportion between the compartments, is in practice not critical. Each compartment volume depends on the volume of constituent to be present therein. In one embodiment, the compartment which accommodates the buffer solution is larger than the compartment/compartments accommodating the glucose solution and is also the compartment in which the solution/solutions from the other compartments is/are mixed with the sodium solution.

In one embodiment, the solution according to the invention is a solution for use as a medicament.

In another embodiment, the medical solution according to the invention is a dialysis solution, e.g. a peritoneal dialysis solution.

The present invention also relates to a method of treatment of dialysis, wherein the solution according to the invention is administered to a patient having a need therefor.

The method for treatment according to a preferred embodiment is peritoneal dialysis.

The term "low levels of glucose degradation products" used herein means that the amount of degradation products from the glucose is so low in the medical solution according to the present invention that it is not more toxic to cultured cells than dialysis solutions according to prior art. In one embodiment of the invention the total sum of the glucose degradation products (5-HMF, 3,4-DGE, glyoxal, methyglyoxal, 3-DG, formaldehyde, and acetaldehyde) in the biocompatible low sodium solution is below 150 µM for fluids with 1.5% glucose, e.g. below 75 µM, below 225 µM for fluids with 2.5% glucose, e.g. below 150 µM, or below 300 µM for fluids with 3.9% glucose, e.g. below 200 µM.

The term "biocompatible solution" used herein means that any biological interaction that is not intended as a part of the treatment does not exist between the solution, and the substances therein, and the living organism, thus not causing toxic or injurious effects on biological function.

The term "sterile" used herein means a condition of a medical device or solution that is free from viable micro-organisms.

The medical solution according to the present invention may also be accomplished by having one or more of the substances in one or more compartments in powder form, which powder is to be mixed with at least one solution to form the final medical solution.

The peritoneal dialysis solution according to the present invention may also comprise other physiologically compatible constituents, e.g. further osmotic agents, such as proteins and peptides, e.g. albumin, as well as antioxidants, such as bisulphite.

The peritoneal dialysis solution of the present invention described above is applicable not only to continuous ambulatory peritoneal dialysis (CAPD) but also to intermittent peritoneal dialysis (IPD), continuous cyclic peritoneal dialysis (CCPD), and automated peritoneal dialysis (APD).

The medical solution according to the present invention has been tested in clinical trials, proving to be hypotensive. The low level of sodium ions in the solution increases the sodium removal from the patient's body, affecting the fluid overload in the body, thus reducing the blood pressure.

### Examples

In all of the Examples below, the pH of the finally mixed sterilised solution is 6.5-7.5 for a lactate containing solution and 7.0-7.8 for a bicarbonate and bicarbonate/lactate containing solution.

### Example 1

Two compartment container with a first compartment 1 comprising sodium ions and a second compartment 2 comprising glucose as well as calcium chloride.

| | | |
|---|---|---|
| Compartment 1 | Volume | 1960 ml |
| | Sodium | 107.37 mM |
| | Calcium | 0.70 mM |
| | Magnesium | 0.27 mM |
| | Chloride | 67.23 mM |
| | Lactate | 31.58 mM |
| | Bicarbonate | 10.5 mM |
| Compartment 2 | Volume | 103 ml |
| | Glucose | 2775 mM |
| | Sodium | 0 mM |
| | Calcium | 19.52 mM |
| | Chloride | 39.04 mM |

It is possible to move a part of the sodium chloride from compartment 1 to compartment 2, if desirable.

After providing the solution comprising sodium ions in the first compartment and the solution comprising glucose in the second compartment, the container, with the two compartments and the solutions therein, is sterilised at a temperature of 121°C. The compartments are delimited from each other during the sterilisation. After sterilisation the sterile contents of compartment 1 and 2 are mixed to constitute the sterile final medical solution.

Final composition when the contents of compartment 1 and 2 are mixed after sterilisation:

| | |
|---|---|
| Volume | 2063 ml |
| Glucose | 138.5 mM (2.5% by weight) |
| Sodium | 102 mM |
| Calcium | 1.64 mM |
| Magnesium | 0.26 mM |
| Chloride | 65.8 mM |
| Lactate | 30 mM |
| Bicarbonate | 10 mM |

### Example 2

Three compartment container in which all compartments contain sodium chloride and calcium chloride in addition, the first compartment 1 comprising other electrolytes and buffering substances and the two further compartments 2 and 3 comprising glucose as well as sodium chloride and calcium chloride.

| | | |
|---|---|---|
| Compartment 1 | Volume | 1960 ml |
| | Sodium | 100.38 mM |
| | Calcium | 0.70 mM |
| | Magnesium | 0.27 mM |
| | Chloride | 60.23 mM |
| | Lactate | 31.58 mM |
| | Bicarbonate | 10.50 mM |
| | | |
| Compartment 2 | Volume | 62 ml |
| | Glucose | 2775.31 mM |
| | Sodium | 1132.27 mM |
| | Calcium | 19.52 mM |
| | Chloride | 1171.32 mM |
| | | |
| Compartment 3 | Volume | 103 ml |
| | Glucose | 2775.31 mM |
| | Sodium | 132.00 mM |
| | Calcium | 19.52 mM |
| | Chloride | 171.05 mM |

It is possible to choose other concentrations of e.g. sodium and chloride in any of the compartments to achieve the desired concentration in the final solution.

The method is accomplished according to Example 1, except that after the sterilisation the contents of compartment 1 are mixed with either the contents of compartment 2 or the contents of compartment 3 or the contents of both compartments 2 and 3.

Final composition when the contents of compartments 1+2, 1+3, or 1+2+3 are mixed after sterilisation:

| 1+2 | |
|---|---|
| Volume | 2022 ml |
| Glucose | 85.1 mM (1.5% by weight) |
| Sodium | 132.0 mM |
| Calcium | 1.28 mM |
| Magnesium | 0.26 mM |
| Chloride | 94.3 mM |
| Lactate | 30.6 mM |
| Bicarbonate | 10.2 mM |
| | |

| 1+3 (according to the invention) | |
|---|---|
| Volume | 2063 ml |
| Glucose | 138.6 mM (2.5% by weight) |
| Sodium | 102 mM |
| Calcium | 1.28 mM |
| Magnesium | 0.26 mM |
| Chloride | 65.8 mM |
| Lactate | 30.0 mM |
| Bicarbonate | 9.98 mM |
| | |

| 1+2+3 | |
|---|---|
| Volume | 2125 ml |
| Glucose | 215.5 mM (3.9% by weight) |
| Sodium | 132.0 mM |
| Calcium | 2.16 mM |
| Magnesium | 0.25 mM |
| Chloride | 98.0 mM |
| Lactate | 29.1 mM |
| Bicarbonate | 9.68 mM |

When mixing compartment 1+2 and 1+2+3 in this example the final sodium concentration is 132 mM, which is the case in conventional solutions. Those concentrations have been chosen to show the flexibility of the three-compartment concept.

### Example 3

Three compartment container with sodium chloride in all three compartments and a first compartment 1 comprising buffering agents and other electrolytes and two further compartments 2 and 3 comprising glucose and calcium chloride.

| | | |
|---|---|---|
| Compartment 1 | Volume | 1960 ml |
| | Sodium | 100.0 mM |
| | Calcium | 0.70 mM |
| | Magnesium | 0.27 mM |
| | Chloride | 59.86 mM |
| | Lactate | 31.58 mM |
| | Bicarbonate | 10.50 mM |
| | | |
| Compartment 2 | Volume | 62 ml |
| | Glucose | 2775.31 mM |
| | Sodium | 719.0 mM |
| | Calcium | 19.52 mM |
| | Chloride | 758.04 mM |
| | | |
| Compartment 3 | Volume | 103 ml |
| | Glucose | 2775.31 mM |
| | Sodium | 140.06 mM |
| | Calcium | 19.52 mM |
| | Chloride | 179.1 mM |

The method is accomplished according to Example 1, except that after the sterilisation the contents of compartment 1 are mixed with either the contents of compartment 2 or the contents of compartment 3 or the contents of both compartments 2 and 3.

Final composition when the contents of compartments 1+2, 1+3, or 1+2+3 are mixed after sterilisation:

| 1+2 | |
|---|---|
| Volume | 2022 ml |
| Glucose | 85.1 mM (1.5% by weight) |
| Sodium | 118.98 mM |
| Calcium | 1.28 mM |
| Magnesium | 0.26 mM |
| Chloride | 81.27 mM |
| Lactate | 30.6 mM |
| Bicarbonate | 10.2 mM |

| 1+3 | |
|---|---|
| Volume | 2063 ml |
| Glucose | 138.6 mM (2.5% by weight) |
| Sodium | 102.0 mM |
| Calcium | 1.64 mM |
| Magnesium | 0.26 mM |
| Chloride | 65.8 mM |
| Lactate | 30.0 mM |
| Bicarbonate | 9.98 mM |
| | |

| 1+2+3 | |
|---|---|
| Volume | 2125 ml |
| Glucose | 215.5 mM (3.9% by weight) |
| Sodium | 120.0 mM |
| Calcium | 2.16 mM |
| Magnesium | 0.25 mM |
| Chloride | 86.0 mM |
| Lactate | 29.1 mM |
| Bicarbonate | 9.68 mM |

### Example 4

Three compartment container where all compartments contain sodium chloride and in addition, first compartment 1 comprising other electrolytes and buffering substances and two further compartments 2 and 3 comprising glucose as well as sodium chloride.

| | | |
|---|---|---|
| Compartment 1 | Volume | 1960 ml |
| | Sodium | 100.38 mM |
| | Calcium | 1.84 mM |
| | Magnesium | 0.27 mM |
| | Chloride | 62.49 mM |
| | Lactate | 42.11 mM |
| Compartment 2 | Volume | 62 ml |
| | Glucose | 2775.31 mM |
| | Sodium | 1132.27 mM |
| | Chloride | 1132.27 mM |
| | | |
| Compartment 3 | Volume | 103 ml |
| | Glucose | 2775.31 mM |
| | Sodium | 132.00 mM |
| | Chloride | 132.00 mM |

It is possible to choose other concentrations of e.g. sodium chloride in any of the compartments to achieve the desired concentration in the final solution.

The method is accomplished according to Example 1, except that after the sterilisation the contents of compartment 1 are mixed with either the contents of compartment 2 or the contents of compartment 3 or the contents of both compartments 2 and 3.

Final composition when the contents of compartments 1+2, 1+3, or 1+2+3 are mixed after sterilisaton:

| 1+2 | |
|---|---|
| Volume | 2022 ml |
| Glucose | 85.1 mM (1.5% by weight) |
| Sodium | 132.0 mM |
| Calcium | 1.78 mM |
| Magnesium | 0.26 mM |
| Chloride | 95.3 mM |
| Lactate | 40.8 mM |

| 1+3 | |
|---|---|
| Volume | 2063 ml |
| Glucose | 138.6 mM (2.5% by weight) |
| Sodium | 102 mM |
| Calcium | 1.75 mM |
| Magnesium | 0.26 mM |
| Chloride | 65.96 mM |
| Lactate | 40.0 mM |
| | |

| 1+2+3 | |
|---|---|
| Volume | 2125 ml |
| Glucose | 215.5 mM (3.9% by weight) |
| Sodium | 132.0 mM |
| Calcium | 1.70 mM |
| Magnesium | 0.25 mM |
| Chloride | 97.1 mM |
| Lactate | 38.8 mM |

When mixing compartments 1+2 and 1+2+3 in this example, the final sodium concentration is 132 mM, which is the case in conventional solutions. These concentrations have been chosen to show the flexibility of the three-compartment concept.

### Example 5

Three compartment container where all compartments contain sodium chloride and in addition, first compartment 1 comprising other electrolytes and buffering substances and two further compartments 2 and 3 comprising glucose as well as sodium chloride.

| | | |
|---|---|---|
| Compartment 1 | Volume | 1960 ml |
| | Sodium | 100.0 mM |
| | Calcium | 1.84 mM |
| | Magnesium | 0.27 mM |
| | Chloride | 62.11 mM |
| | Lactate | 42.11 mM |
| Compartment 2 | Volume | 62 ml |
| | Glucose | 2775.31 mM |
| | Sodium | 719.00 mM |
| | Chloride | 719.00 mM |
| | | |
| Compartment 3 | Volume | 103 ml |
| | Glucose | 2775.31 mM |
| | Sodium | 140.06 mM |
| | Chloride | 140.06 mM |

It is possible to choose other concentrations of e.g. sodium chloride in any of the compartments to achieve the desired concentration in the final solution.

The method is accomplished according to Example 1, except that after the sterilisation the contents of compartment 1 are mixed with either the contents of compartment 2 or the contents of compartment 3 or the contents of both compartments 2 and 3.

Final composition when the contents of compartments 1+2, 1+3, or 1+2+3 are mixed after sterilisation:

| 1+2 | |
|---|---|
| Volume | 2022 ml |
| Glucose | 85.1 mM (1.5% by weight) |
| Sodium | 118.98 mM |
| Calcium | 1.78 mM |
| Magnesium | 0.26 mM |
| Chloride | 82.25 mM |
| Lactate | 40.8 mM |

| 1+3 | |
|---|---|
| Volume | 2063 ml |
| Glucose | 138.6 mM (2.5% by weight) |
| Sodium | 102 mM |
| Calcium | 1.75 mM |
| Magnesium | 0.26 mM |
| Chloride | 66.00 mM |
| Lactate | 40.0 mM |
| | |

| 1+2+3 | |
|---|---|
| Volume | 2125 ml |
| Glucose | 215.5 mM (3.9% by weight) |
| Sodium | 120.00 mM |
| Calcium | 1.70 mM |
| Magnesium | 0.25 mM |
| Chloride | 85.05 mM |
| Lactate | 38.8 mM |

### Example 6

Three compartment container where all compartments contain sodium chloride and in addition, first compartment 1 comprising other electrolytes and buffering substances and two further compartments 2 and 3 comprising glucose as well as sodium chloride and calcium chloride.

| | | |
|---|---|---|
| Compartment 1 | Volume | 1960 ml |
| | Sodium | 100.0 mM |
| | Calcium | 0.70 mM |
| | Magnesium | 0.27 mM |
| | Chloride | 59.86 mM |
| | Bicarbonate | 42.08 mM |
| | | |
| Compartment 2 | Volume | 62 ml |
| | Glucose | 2775.31 mM |
| | Sodium | 719.0 mM |
| | Calcium | 19.52 mM |
| | Chloride | 758.04 mM |
| Compartment 3 | Volume | 103 ml |
| | Glucose | 2775.31 mM |
| | Sodium | 140.06 mM |
| | Calcium | 19.52 mM |
| | Chloride | 179.1 mM |

It is possible to choose other concentrations of e.g. sodium chloride in any of the compartments to achieve the desired concentration in the final solution.

The method is accomplished according to Example 1, except that after the sterilisation the contents of compartment 1 are mixed with either the contents of compartment 2 or the contents of compartment 3 or the contents of both compartments 2 and 3.

Final composition when the contents of compartments 1+2, 1+3, or 1+2+3 are mixed after sterilisation:

| | |
|---|---|
| 1+2 | |
| Volume | 2022 ml |
| Glucose | 85.1 mM (1.5% by weight) |
| Sodium | 118.98 mM |
| Calcium | 1.28 mM |
| Magnesium | 0.26 mM |
| Chloride | 81.27 mM |
| Bicarbonate | 40.07 mM |
| | |
| 1+3 | |
| Volume | 2063 ml |
| Glucose | 138.6 mM (2.5% by weight) |
| Sodium | 102 mM |
| Calcium | 1.64 mM |
| Magnesium | 0.26 mM |
| Chloride | 65.81 mM |
| Bicarbonate | 33.98 mM |

| 1+2+3 | |
|---|---|
| Volume | 2125 ml |
| Glucose | 215.5 mM (3.9% by weight) |
| Sodium | 120.0 mM |
| Calcium | 2.16 mM |
| Magnesium | 0.25 mM |
| Chloride | 86.01 mM |
| Bicarbonate | 38.81 mM |

### Example 7

Three compartment container where all compartments contain sodium chloride and in addition, first compartment 1 comprising other electrolytes and buffering substances and two further compartments 2 and 3 comprising glucose as well as sodium chloride.

| | | |
|---|---|---|
| Compartment 1 | Volume | 1900 ml |
| | Sodium | 64.0 mM |
| | Calcium | 1.42 mM |
| | Magnesium | 0.27 mM |
| | Chloride | 25.27 mM |
| | Lactate | 42.11 mM |
| | | |
| Compartment 2 | Volume | 79.5 ml |
| | Glucose | 2775.31 mM |
| | Sodium | 327.0 mM |
| | Chloride | 327.0 mM |
| | | |
| Compartment 3 | Volume | 134 ml |
| | Glucose | 2775.31 mM |
| | Sodium | 39.6 mM |
| | Chloride | 39.6 mM |

It is possible to choose other concentrations of e.g. sodium chloride in any of the compartments to achieve the desired concentration in the final solution. Lactate can be exchanged either completely or partially with another buffering substance, e.g. bicarbonate.

The method is accomplished according to Example 1, except that after the sterilisation the contents of compartment 1 are mixed with either the contents of compartment 2 or the contents of compartment 3 or the contents of both compartments 2 and 3.

Final composition when the contents of compartments 1+2, 1+3, or 1+2+3 are mixed after sterilisation:

| 1+2 | |
|---|---|
| Volume | 1979.5 ml |
| Glucose | 111.46 mM (2.0% by weight) |
| Sodium | 114.98 mM |
| Calcium | 1.36 mM |
| Magnesium | 0.26 mM |
| Chloride | 37.38 mM |
| Lactate | 40.42 mM |

| 1+3 | |
|---|---|
| Volume | 2034 ml |
| Glucose | 182.8 mM (3.3% by weight) |
| Sodium | 101.7 mM |
| Calcium | 1.33 mM |
| Magnesium | 0.25 mM |
| Chloride | 26.21 mM |
| Lactate | 39.34 mM |

| 1+2+3 | |
|---|---|
| Volume | 2114 ml |
| Glucose | 280.4 mM (5.1% by weight) |
| Sodium | 110.3 mM |
| Calcium | 1.28 mM |
| Magnesium | 0.24 mM |
| Chloride | 37.5 mM |
| Lactate | 37.86 mM |

### Example 8

Three compartment container where all compartments contain sodium chloride and in addition, first compartment 1 comprising other electrolytes and buffering substances and two further compartments 2 and 3 comprising glucose as well as sodium chloride.

| | | |
|---|---|---|
| Compartment 1 | Volume | 1900 ml |
| | Sodium | 64.8 mM |
| | Calcium | 1.42 mM |
| | Magnesium | 0.27 mM |
| | Chloride | 26.07 mM |
| | Lactate | 42.11 mM |
| | | |
| Compartment 2 | Volume | 79.5 ml |
| | Glucose | 2775.31 mM |
| | Sodium | 307.9 mM |
| | Chloride | 307.9 mM |
| | | |
| Compartment 3 | Volume | 100 ml |
| | Glucose | 2775.31 mM |
| | Sodium | 8.72 mM |
| | Chloride | 8.72 mM |

It is possible to choose other concentrations of e.g. sodium chloride in any of the compartments to achieve the desired concentration in the final solution.

The method is accomplished according to Example 1, except that after the sterilisation the contents of compartment 1 are mixed with either the contents of compartment 2 or the contents of compartment 3 or the contents of both compartments 2 and 3.

Final composition when the contents of compartments 1+2, 1+3, or 1+2+3 are mixed after sterilisation:

| 1+2 | | |
|---|---|---|
| Volume | 1980 ml | |
| Glucose | 111.46 mM | (2.0% by weight) |
| Sodium | 114.98 mM | |
| Calcium | 1.36 mM | |
| Magnesium | 0.26 mM | |
| Chloride | 37.38 mM | |
| Lactate | 40.42 mM | |

| | | |
|---|---|---|
| 1+3 | | |
| Volume | 2000 ml | |
| Glucose | 138.8 mM | (2.5% by weight) |
| Sodium | 102 mM | |
| Calcium | 1.35 mM | |
| Magnesium | 0.25 mM | |
| Chloride | 25.20 mM | |

| Lactate | 40.0 mM | |
|---|---|---|
| | | |
| 1+2+3 | | |
| Volume | 2080 ml | |
| Glucose | 239.6 mM | (4.3% by weight) |
| Sodium | 109.9 mM | |
| Calcium | 1.30 mM | |
| Magnesium | 0.24 mM | |
| Chloride | 36.01 mM | |
| Lactate | 38.48 mM | |

### Example 9

Three compartment container where all compartments contain sodium chloride and in addition, first compartment 1 comprising other electrolytes and buffering substances and two further compartments 2 and 3 comprising glucose as well as sodium chloride and calcium chloride.

| | | |
|---|---|---|
| Compartment 1 | Volume | 1900 ml |
| | Sodium | 64.8 mM |
| | Calcium | 0.7 mM |
| | Magnesium | 0.27 mM |
| | Chloride | 24.7 mM |
| | Lactate | 31.58 mM |
| | Bicarbonate | 10.5 mM |
| | | |
| Compartment 2 | Volume | 79.5 ml |
| | Glucose | 2775.31 mM |
| | Sodium | 327.0 mM |
| | Calcium | 17.5 mM |
| | Chloride | 362.0 mM |
| | | |
| Compartment 3 | Volume | 134 ml |
| | Glucose | 2775.31 mM |
| | Sodium | 39.6 mM |
| | Calcium | 15.2 mM |
| | Chloride | 70.0 mM |

It is possible to choose other concentrations of e.g. sodium chloride in any of the compartments to achieve the desired concentration in the final solution.

The method is accomplished according to Example 1, except that after the sterilisation the contents of compartment 1 are mixed with either the contents of compartment 2 or the contents of compartment 3 or the contents of both compartments 2 and 3.

Final composition when the contents of compartments 1+2, 1+3, or 1+2+3 are mixed after sterilisation:

| 1+2 | |
|---|---|
| Volume | 1980 ml |
| Glucose | 111.46 mM (2.0% by weight) |
| Sodium | 115.72 mM |
| Calcium | 1.37 mM |
| Magnesium | 0.26 mM |
| Chloride | 38.2 mM |
| Lactate | 30.31 mM |
| Bicarbonate | 10.1 mM |
| | |

| 1+3 | |
|---|---|
| Volume | 2034 ml |
| Glucose | 182.8 mM (3.3% by weight) |
| Sodium | 102.5 mM |
| Calcium | 1.66 mM |
| Magnesium | 0.25 mM |
| Chloride | 27.6 mM |
| Lactate | 29.5 mM |
| Bicarbonate | 9.81 mM |
| | |

| 1+2+3 | |
|---|---|
| Volume | 2114 ml |
| Glucose | 280.4 mM (5.1% by weight) |
| Sodium | 111.0 mM |
| Calcium | 2.25 mM |
| Magnesium | 0.24 mM |
| Chloride | 40.22 mM |
| Lactate | 28.4 mM |
| Bicarbonate | 9.44 mM |

### Example 10

Three compartment container where all compartments contain sodium chloride and in addition, first compartment 1 comprising other electrolytes and buffering substances and two further compartments 2 and 3 comprising glucose as well as sodium chloride.

| | | |
|---|---|---|
| Compartment 1 | Volume | 1900 ml |
| | Sodium | 64.8 mM |
| | Calcium | 0.7 mM |
| | Magnesium | 0.27 mM |
| | Chloride | 24.7 mM |
| | Lactate | 31.58 mM |
| | Bicarbonate | 10.5 mM |
| | | |
| Compartment 2 | Volume | 79.5 ml |
| | Glucose | 2775.31 mM |
| | Sodium | 307.9 mM |
| | Calcium | 17.55 mM |
| | Chloride | 343.0 mM |
| | | |
| Compartment 3 | Volume | 100 ml |
| | Glucose | 2775.31 mM |
| | Sodium | 8.72 mM |
| | Calcium | 15.2 mM |
| | Chloride | 48.12 mM |

It is possible to choose other concentrations of e.g. sodium chloride in any of the compartments to achieve the desired concentration in the final solution.

The method is accomplished according to Example 1, except that after the sterilisation the contents of compartment 1 are mixed with either the contents of compartment 2 or the contents of compartment 3 or the contents of both compartments 2 and 3.

Final composition when the contents of compartments 1+2, 1+3, or 1+2+3 are mixed after sterilisation:

| 1+2 | |
|---|---|
| Volume | 1980 ml |
| Glucose | 111.46 mM (2.0% by weight) |
| Sodium | 114.95 mM |
| Calcium | 1.38 mM |
| Magnesium | 0.26 mM |
| Chloride | 37.4 mM |
| Lactate | 30.31 mM |
| Bicarbonate | 10.1 mM |
| | |

| 1+3 | |
|---|---|
| Volume | 2000 ml |
| Glucose | 138.8 mM (2.5% by weight) |
| Sodium | 102 mM |
| Calcium | 1.65 mM |
| Magnesium | 0.25 mM |
| Chloride | 25.8 mM |
| Lactate | 30.0 mM |
| Bicarbonate | 9.98 mM |
| | |

| 1+2+3 | |
|---|---|
| Volume | 2080 ml |
| Glucose | 239.6 mM (4.3% by weight) |
| Sodium | 109.9 mM |
| Calcium | 2.26 mM |
| Magnesium | 0.24 mM |
| Chloride | 37.95 mM |
| Lactate | 28.85 mM |
| Bicarbonate | 9.59 mM |

Both independently from and in combination with the preceding embodiments and description, the present invention further comprises the following embodiments:
1. A medical solution comprising sodium ions in a concentration of 90 to 125 mM, glucose in a concentration of 1% to 5% by weight, and a low level of glucose degradation products, wherein the solution is sterile and has a pH of 6.5 to 8.0.
2. The medical solution according to embodiment 1, wherein the total sum of the glucose degradation products is below 150 µM for fluids with 1.5% by weight of glucose, below 225 µM for fluids with 2.5% by weight of glucose, or below 300 µM for fluids with 3.9% by weight of glucose.
3. The medical solution according to embodiment 2, wherein the total sum of the glucose degradation products is below 75 µM for fluids with 1.5% by weight of glucose, below 150 µM for fluids with 2.5% by weight of glucose, and below 200 µM for fluids with 3.9% by weight of glucose.
4. The medical solution according to any one of the preceding embodiments, wherein the concentration of sodium ions is 100 to 115 mM.
5. The medical solution according to any one of the preceding embodiments, wherein the glucose concentration is 1.5% to 4% by weight.
6. The medical solution according to any one of the preceding embodiments, wherein the pH is 7.0 to 7. 8.
7. The medical solution according to any one of the preceding embodiments, wherein it contains, after mixing, a sodium concentration of 102 mM to 115 mM and a glucose concentration of 2.0%, 2.5%, or 4.3% by weight and a pH of 7.4.
8. The medical solution according to any one of the preceding embodiments, wherein the concentration of sodium ions is 102 mM and the pH is 7.4.
9. The medical solution according to any one of the preceding embodiments, wherein it also comprises bicarbonate ions in a concentration of 5 to 45 mM, or lactate in a concentration of 5 to 45 mM, or a combination of both where the total concentration of bicarbonate and lactate does not exceed 45 mM.
10. The medical solution according to embodiment 9, wherein the bicarbonate concentration is 25 to 40 mM.
11. The medical solution according to embodiment 9, wherein the lactate concentration is 25 to 40 mM.
12. The medical solution according to any one of the preceding embodiments, wherein the solution also comprises other electrolytes.
13. The medical solution according to embodiment 12, wherein said other electrolytes are one or more of potassium, calcium, and magnesium.
14. A method for preparing a medical solution according to any one of the preceding embodiments, comprising the steps of
   a) providing a solution comprising sodium ions in a first compartment of a container,
   b) providing a solution comprising glucose in at least one further compartment of the container,
   c) sterilisation of the container with said compartments and the solutions therein, wherein each compartment is delimited from the other/others during the sterilisation,
   d) mixing of the sterilised solution in the first compartment with the sterilised solution in at least one of said further compartments to the final medical solution.
15. The method according to embodiment 14, wherein the pH in the at least one further compartment including glucose is 2 to 5.
16. The method according to embodiment 14 or 15, wherein the glucose concentration in the at least one further compartment is provided to be above 10%.
17. The method according to embodiment 16, wherein said glucose concentration is above 20% by weight.
18. The method according to embodiment 17, wherein said glucose concentration is above 40% by weight.
19. The method according to any one of embodiments 14 to 18, wherein sodium ions are also provided within the at least one further compartment containing glucose.
20. The method according to any one of embodiments 14 to 19, wherein when the medical solution comprises bicarbonate ions and/or lactate, the bicarbonate ions and/or lactate are/is provided within the first compartment of said container.
21. The method according to any one of embodiments 14 to 20, wherein the sterilisation is heat sterilisation at a temperature of at least 100°C.
22. The method according to embodiment 21, wherein said temperature is above 120°C.
23. The method according to any one of embodiments 14 to 22, wherein the container comprises two compartments.
24. The method according to any one of embodiments 14 to 22, wherein the container comprises at least three compartments.
25. A container for preparation of the medical solution according to any one of embodiments 1 to 13, comprising a first compartment containing a solution comprising sodium ions and at least one further compartment containing a solution comprising glucose.
26. The container according to embodiment 25, wherein the container comprises two compartments.
27. The container according to embodiment 25, wherein the container comprises at least three compartments.
28. The container according to embodiment 25, wherein the first compartment further also contains bicarbonate ions and/or lactate.
29. A solution according to any one of embodiments 1 to 13 for use as a medicament.
30. Use of a medical solution according to any one of embodiments 1 to 13 for manufacture of a medicament for dialysis.
31. Use according to embodiment 30, wherein the dialysis is peritoneal dialysis.
32. A method for treatment of dialysis, wherein the solution according to any one of embodiments 1 to 13 is administered to a patient having a need therefor.
33. The method according to embodiment 32, wherein the dialysis is peritoneal dialysis.

## Claims

1. A container for preparation of a peritoneal dialysis solution, the container comprising a first compartment containing a first solution comprising sodium ions and at least a second compartment containing a second solution comprising glucose, wherein mixing of the first solution with the at least second solution results in a final solution comprising sodium ions in a concentration of 100 to 115 mM, glucose in a concentration of 1% to 5% by weight, and a low level of glucose degradation products.

2. The container of claim 1, wherein the final solution is sterile and has a pH of 6.5 to 8. 0, wherein the final solution preferably has a pH of 6.5 to 7,5 for a lactate containing solution and 7.0 to 7.8 for a bicarbonate or bicarbonate and lactate containing solution.

3. The container according to claim 1 or 2, wherein the total sum of the glucose degradation products from the group consisting of 5-Hydroxymethylfuraldehyde, 3,4-Dideoxyglucosone, Glyoxal, Methylglyoxal, 3-Deoxyglucosoneformaldehyde, Formaldehyde and Acetaldehyde in the final solution is below 150 µM for fluids with 1.5% by weight of glucose, below 225 µM for fluids with 2.5% by weight of glucose, or below 300 µM for fluids with 3.9% by weight of glucose.

4. The container according to claim 3, wherein the total sum of the glucose degradation products from the group consisting of 5-Hydroxymethylfuraldehyde, 3,4-Dideoxyglucosone, Glyoxal, Methylglyoxal, 3-Deoxyglucosoneformaldehyde, Formaldehyde and Acetaldehyde in the final solution in the final solution is below 75 µM for fluids with 1.5% by weight of glucose, below 150 µM for fluids with 2.5% by weight of glucose, and below 200 µM for fluids with 3.9% by weight of glucose.

5. The medical solution according to any one of the preceding claims, wherein the glucose concentration in the final solution is 1.5% to 4% by weight and/or wherein the pH of the final solution is 7.0 to 7. 8.

6. The container according to any one of the preceding claims, wherein the final solution comprises a sodium concentration of 102 mM to 115 mM and a glucose concentration of 2.0%, 2.5%, or 4.3% by weight and a pH of 7.4.

7. The container according to any one of the preceding claims, wherein the concentration of sodium ions in the final solution is 102 mM and the pH is 7.4.

8. The container according to any one of the preceding claims, wherein the final solution comprises bicarbonate ions and/or lactate, wherein the bicarbonate ions and/or lactate are/is preferably provided within the first compartment, and/or wherein the final solution preferably comprises bicarbonate ions in a concentration of 5 to 45 mM, or lactate in a concentration of 5 to 45 mM, or a combination of both where the total concentration of bicarbonate and lactate does not exceed 45 mM, wherein the bicarbonate concentration preferably is 25 to 40 mM and/or wherein the lactate concentration preferably is 25 to 40 mM.

9. The container according to any one of the preceding claims, wherein the final solution also comprises other electrolytes, wherein said other electrolytes preferably are one or more of potassium, calcium, and magnesium.

10. The container according to any one of the preceding claims, wherein the pH in the at least one second compartment including glucose is 2 to 5 and/or wherein the glucose concentration in the at least one second compartment is provided to be above 10%, preferably above 20% by weight, prefereably above 40% by weight.

11. The container according to any one of the preceding claims, wherein sodium ions are also provided within the at least one second compartment containing glucose.

12. The container according to any one of the preceding claims, wherein the container comprises two compartments.

13. The container according to any one of claims 1 to 11, wherein the container comprises at least three compartments.

14. A method for preparing a peritoneal dialysis solution using a container according to any one of the preceding claims, comprising the steps of
a) providing a first solution comprising sodium ions in a first compartment of a container,
b) providing a second solution comprising glucose in at least one second compartment of the container,
c) sterilisation of the container with said compartments and the solutions therein, wherein each compartment is delimited from the other/others during the sterilisation,
d) mixing of the sterilised first solution with the at least one sterilised second solution to the final medical solution.

15. The method according to claim 12, wherein the sterilisation is heat sterilisation at a temperature of at least 100°C, wherein said temperature is preferably above 120°C.
